(19) Europäisches Patentamt
European Patent Office
Office européen des brevets

(11) Publication number: **0 171 283**
**A2**

(12) **EUROPEAN PATENT APPLICATION**

(21) Application number: **85305600.0**

(22) Date of filing: **06.08.85**

(51) Int. Cl.⁴: **C 12 P 17/10**, C 07 D 209/80, C 07 D 209/14 // (C12P17/10, C12R1:89)

(30) Priority: **08.08.84 US 638847**

(43) Date of publication of application: **12.02.86 Bulletin 86/7**

(84) Designated Contracting States: **AT BE CH DE FR GB IT LI NL SE**

(71) Applicant: **UNIVERSITY OF HAWAII, 2545 The Mall, Honolulu Hawaii 96822 (US)**

(72) Inventor: **Moore, Richard Elliott, 4494 Pahow Avenue, Honolulu Hawaii 96816 (US)**
Inventor: **Patterson, Gregory Matthew Leon, 1910 Aleo Street, Honolulu Hawaii 96822 (US)**

(74) Representative: **Hudson, Christopher Mark et al, Erl Wood Manor, Windlesham Surrey GU20 6PH (GB)**

(54) **Hapalindoles.**

(57) New alkaloids called hapalindoles, which are antibacterial and antifungal agents, and methods of preparing these alkaloids by culturing the blue-green alga Hapalosiphon fontinalis ATCC 39694, are provided.

EP 0 171 283 A2

# HAPALINDOLES

This invention relates to new antibacterial agents. In particular, this invention provides useful new alkaloids, called hapalindoles A-I, which can be prepared by culturing the blue-green alga Hapalosiphon fontinalis ATCC 39694. The new alkaloids of this invention have been assigned the structures shown in formulae 1-9

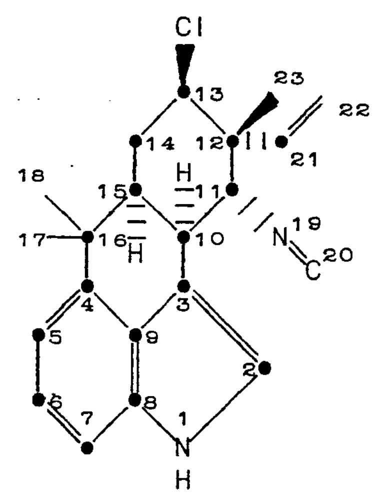

Hapalindole A (1)

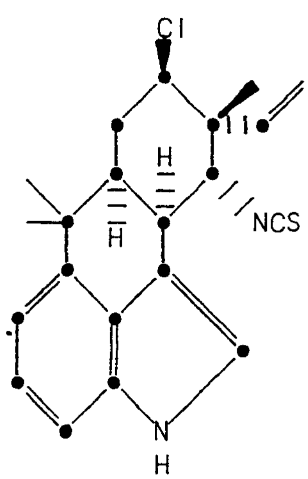

Hapalindole B (2)

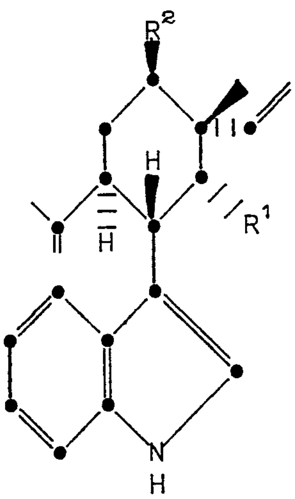

| | $R^1$ | $R^2$ |
|---|---|---|
| Hapalindole C (3) | NC | H |
| Hapalindole D (4) | NCS | H |
| Hapalindole E (5) | NC | Cl |
| Hapalindole F (6) | NCS | Cl |

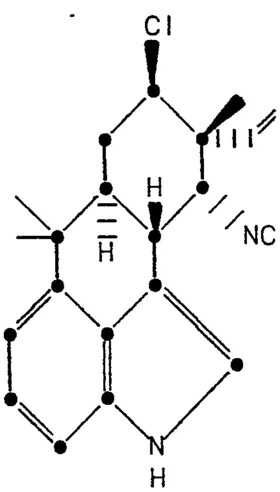

Hapalindole G (7)

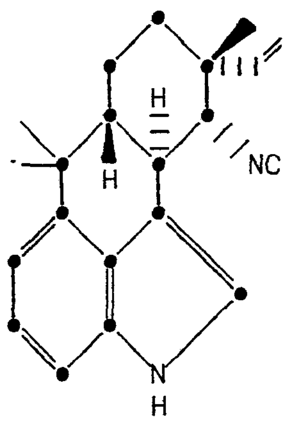

Hapalindole H (8)

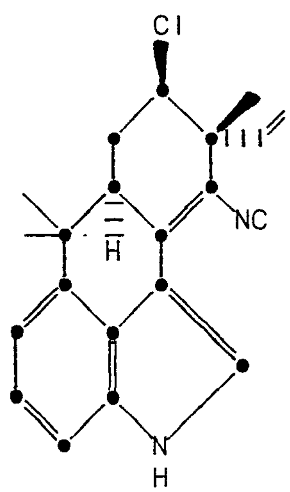

Hapalindole I (9)

The hapalindoles of this invention are produced by cultivating a new strain of the blue-green alga

Hapalosiphon fontinalis (Ag.) Bornet (Stigonemataceae). This new strain, which was isolated from soil samples collected in the Marshall Islands by repeated subculture on solidified media, was given the strain number V-3-1. It has been deposited and made part of the stock culture collection of The American Type Culture Collection, 12301 Parklawn Drive, Rockville, Maryland 20852, from which it is available to the public under the accession number ATCC 39694.

As is the case with other organisms, the characteristics of Hapalosiphon fontinalis ATCC 39694 are subject to variation. For example, recombinants, variants or mutants of the ATCC 39694 strain may be obtained by treatment with various known physical and chemical mutagens, such as ultraviolet rays, X-rays, gamma rays, and N-methyl-N'nitro-N-nitrosoguanidine. All natural and induced variants, mutants and recombinants of Hapalosiphon fontinalis ATCC 39694 which retain the characteristic of producing a hapalindole compound may be used in this invention.

The hapalindole alkaloids of this invention are prepared by culturing a strain of Hapalosiphon fontinalis which produces these compounds under submerged aerobic conditions in a suitable culture medium until substantial antibiotic activity is produced. Other culture techniques, such as surface growth on solidified media, can also be used to produce these compounds. The culture medium used to grow Hapalosiphon fontinalis ATCC 39694 can be any one of a number of media. Economy in production, optimal yield, and ease

of product isolation are factors to consider when choosing the carbon sources and nitrogen sources to be used. Among the nutrient inorganic salts which can be incorporated in the culture media are the customary soluble salts capable of yielding iron, potassium, sodium, magnesium, calcium, ammonium, chloride, carbonate, phosphate, sulfate, nitrate, and like ions.

Essential trace elements necessary for the growth and development of the organism should also be included in the culture medium. Such trace elements commonly occur as impurities in other constituents of the medium in amounts sufficient to meet the growth requirements of the organism. It may be necessary to add small amounts (i.e. 0.2 ml/L) of an antifoam agent such as polypropylene glycol (M.W. about 2000) to large-scale fermentation media if foaming becomes a problem.

For production of substantial quantities of the hapalindoles, submerged aerobic cultivation in tanks can be used. Small quantities may be obtained by shake-flask culture. Because of the time lag in antibiotic production commonly associated with inoculation of large tanks with the organism, it is preferable to use a vegetative inoculum. The vegetative inoculum is prepared by inoculating a small volume of culture medium with the spore or akinete-containing form or fragments of the vegetative trichome of the organism to obtain a fresh, actively growing culture of the organism. The vegetative inoculum is then transferred to a larger tank. The medium used for the vegetative inoculum can

be the same as that used for larger fermentations, but other media can also be used.

H. fontinalis ATCC 39694 can be grown at temperatures between about 20° and about 30°C. The hapalindole compounds are produced at a temperature of about 24°C and an incident illumination intensity of 330 microEinsteins-$m^{-2}$-$sec^{-1}$. Light intensities somewhat higher or lower can also be used to produce these compounds.

As is customary in aerobic submerged culture processes of this type, $CO_2$ in sterile air is bubbled through the culture medium. For efficient production of the hapalindoles, the percent of $CO_2$ should be about 1% (at 24°C. and one atmosphere of pressure).

Hapalindole production can be followed during the cultivation by testing samples of the broth against organisms known to be sensitive to these antibiotics. One useful assay organism is Staphylococcus aureus.

Following their production under submerged aerobic cultivation conditions, the hapalindoles can be recovered from the cultivation medium by methods used in this art. Recovery is generally accomplished by initially filtering the culture medium to separate the algal cells and then freeze drying the separated cells. The freeze fried alga can be be extracted with a suitable solvent such as isopropanol, dichloromethane, or ethyl acetate. The alkaloids can be separated by subjecting this extract to gel filtration and silica-gel chromatography. The alkaloids can be purified by high-performance liquid chromatography (HPLC).

The hapalindoles of this invention inhibit the growth of various pathogenic bacteria, especially gram-positive bacteria. Table I summarizes the minimal inhibitory concentrations (MIC's) at which the compounds inhibit certain organisms, as determined by standard agar-dilution assays.

Table I: In Vitro Antibacterial Activity Of Hapalindoles

| Organism | MIC (mcg/ml) Compound No. 1 | 2 | 3 | 4 | 5 | 6 | 7 | 8 |
|---|---|---|---|---|---|---|---|---|
| *Staphylococcus aureus* X1.1 | 4 | 2 | 16 | 4 | 4 | 16 | 64 | >64 |
| *Staphylococcus aureus* V41 | 4 | 2 | 16 | 4 | 2 | >64 | 64 | >64 |
| *Staphylococcus* aureus X400 | 4 | 2 | 16 | 8 | 4 | 32 | 64 | >64 |
| *Staphylococcus aureus* S13E | 4 | 2 | 8 | 4 | 2 | 32 | 64 | >64 |
| *Staphylococcus epidermidis* EPI1 | 2 | 2 | 8 | 8 | 2 | 4 | 8 | 32 |
| *Staphylococcus epidermidis* 222 | 0.06 | 2 | 16 | 4 | 2 | 4 | 16 | 32 |
| *Streptococcus pyogenes* C203 | 2 | 64 | >64 | >64 | >64 | >64 | 16 | 32 |
| *Streptococcus pneumoniae* Park 1 | 16 | 64 | >64 | >64 | >64 | >64 | 16 | 32 |
| *Streptococcus faecium* ATCC 9790 | 16 | 4 | >64 | 8 | 64 | 64 | >64 | >64 |
| *Streptococcus sp*. group D 2041 | 16 | 8 | >64 | 8 | >64 | 16 | >64 | >64 |
| *Haemophilus influenzae* C.L. | 4 | >64 | 16 | >64 | 4 | >64 | 64 | >64 |
| *Haemophilus influenzae* 76 | 4 | >64 | 16 | >64 | 4 | >64 | 64 | >64 |
| *Escherichia coli* N10 | 4 | >64 | >64 | >64 | 16 | >64 | >64 | >64 |
| *Escherichia coli* EC14 | 8 | >64 | >64 | >64 | 16 | >64 | >64 | >64 |
| *Escherichia coli* TEM | 4 | >64 | 64 | >64 | >64 | >64 | >64 | >64 |

Table I (continued)

| Organism | MIC (mcg/ml) | | | | | | | |
|---|---|---|---|---|---|---|---|---|
| | Compound No. | | | | | | | |
| | 1 | 2 | 3 | 4 | 5 | 6 | 7 | 8 |
| Klebsiella pneumoniae X26 | 4 | >64 | >64 | >64 | >64 | >64 | 64 | >64 |
| Klebsiella pneumoniae X68 | >64 | >64 | >64 | >64 | >64 | >64 | >64 | >64 |
| Klebsiella pneumoniae KAE | >64 | >64 | >64 | >64 | >64 | >64 | >64 | >64 |
| Salmonella sp. X514 | 8 | >64 | >64 | >64 | >64 | >64 | >64 | >64 |
| Shigella sonnei N9 | 8 | >64 | >64 | >64 | >64 | >64 | >64 | >64 |
| Proteus morganii PR15 | 32 | >64 | >64 | >64 | 32 | >64 | >64 | >64 |
| Acinetobacter calcoaceticus AC12 | 2 | >64 | 2 | >64 | >64 | >64 | 16 | 16 |

The hapalindoles of this invention also inhibit the growth of pathogenic fungi. Table II summarizes the MIC's at which the compounds inhibit test organisms, as determined by standard disc-plate assays.

Table II: In Vitro Antifungal Activity of Hapalindoles

| | MIC mcg/ml | |
|---|---|---|
| Compound | Candida albicans | Trichophyton mentagrophytes |
| 1 | 1.25 | 1.25 |
| 2 | >20 | >20 |
| 3 | 0.625 | 0.625 |
| 4 | >20 | >20 |
| 5 | 0.312 | 0.625 |
| 6 | >20 | >20 |
| 7 | 10 | 2.5 |
| 8 | 10 | 1.25 |

Example 1

Culture of *Hapalosiphon fontinalis* ATCC 39694

*Hapalosiphon fontinalis* strain V-3-1 (ATCC 39694) was cultured in 25-L glass bottles containing an inorganic medium having the following composition:

| Ingredient | Amount |
|---|---|
| $NaNO_3$ | 200 mg/L |
| $NH_4Cl$ | 10 mg/L |
| $K_2HPO_4 \cdot 3H_2O$ | 65 mg/L |
| $MgSO_4 \cdot 7H_2O$ | 50 mg/L |
| $CaCl_2 \cdot 2H_2O$ | 13 mg/L |
| 3-(N-morpholino)-propanesulfonic acid | 627 mg/L |
| Minor elements solution[a] | 1 mL/L |
| Trace elements solution[b] | 3/25 (0.12) mL/L |

Prior to autoclaving, the pH of the complete medium is adjusted to 7 with sodium hydroxide.

[a]Minor Elements Solution:

| Ingredient | Amount |
|---|---|
| $FeCl_3 \cdot 6H_2O$ | 0.54 g/L |
| $Na_2EDTA$ | 3.0 g/L |
| $H_3BO_3$ | 0.62 g/L |
| $MnCl_2 \cdot 4H_2O$ | 1.4 g/L |
| $ZnCl_2$ | 0.10 g/L |
| $CoCl_2 \cdot 6H_2O$ | 5 mg/L |
| $CuCl_2 \cdot 2H_2O$ | 34 mcg/L |

[b]Trace Elements Solution:

| Ingredient | Amount (mg/10 L of 0.1 N $H_2SO_4$) |
|---|---|
| $MoO_3$ (85%) | 176.4 |
| $NH_4VO_3$ | 229.6 |
| $Cr_2K_2(SO_4)_4 \cdot 24H_2O$ | 960.2 |
| $NiSO_4 \cdot 6H_2O$ | 447.8 |
| $Co(NO_3)_2 \cdot 6H_2O$ | 493.8 |
| $Na_2WO_4 \cdot 2H_2O$ | 179.4 |
| $Al_2(SO_4)_3$ | 317.1 |
| $As_2O_3$ | 66.1 |
| $CdCl_2$ | 81.5 |
| $SrSO_4$ | 104.9 |
| $HgCl_2$ | 67.7 |
| $PbCl_2$ | 67.1 |
| LiCl | 305.5 |
| $Rb_2SO_4$ | 78.1 |
| NaBr | 64.4 |
| KI | 65.4 |
| NaF | 110.5 |
| $Na_2SeO_4$ | 119.4 |
| $Be(NO_3)_2 \cdot 3H_2O$ | 1037.0 |

Cultures were illuminated continuously at an incident intensity of 330 microEinsteins-$m^{-2}$-$sec^{-1}$ from banks of cool-white fluorescent tubes. Cultures were vigorously aerated with 1% $CO_2$ in air and incubated at 24 ± 1°C. Alga was cultured for 24 days and then was

harvested by filtration; yields typically were 0.4-0.5 g dry weight of cells per liter of culture.

## Example 2

### A. Isolation of Hapalindoles

Freeze-dried alga prepared as in Example 1 (360 g) was extracted with 1:1 i-PrOH/$CH_2Cl_2$. The oily extract (15.1 g) was subjected to gel filtration on Sephadex LH-20 with 1:1 i-PrOH/$CH_2Cl_2$ and then to rapid chromatography on silica gel (tlc grade), eluting with hexane, 1:1 hexane/$CH_2Cl_2$, $CH_2Cl_2$, $CH_2Cl_2$/EtOAc, EtOAc, and EtOAC/EtOH. The fraction eluted with 1:1 hexane/$CH_2Cl_2$ was purified by HPLC on Whatman Partisil with 1:1 hexane/$CH_2Cl_2$ to give [after crystallization from $CH_2Cl_2$ and sublimation at 125° (0.1 mm)] 2.1 g (0.58%) of hapalindole A (1).

The remaining hapalindoles were isolated in smaller amounts from the Partisil column. The nine alkaloids were eluted in the following order: 4, 6, 2, 1, 5, 3, 8, 7 and 9.

### B. Characteristics of Hapalindole A (1)

Empirical formula: $C_{21}H_{23}N_2Cl$;
mp: 160-167° (dec);
$[\alpha]_D$: -78° (c 1.2, $CH_2Cl_2$);
UV $\lambda_{max}$(ethanol): 222 nm (ε38,000), 280 (7000), 291 (5800)

IR ($CHCl_3$): 2145 $cm^{-1}$.

High resolution EIMS m/z: 338.1595

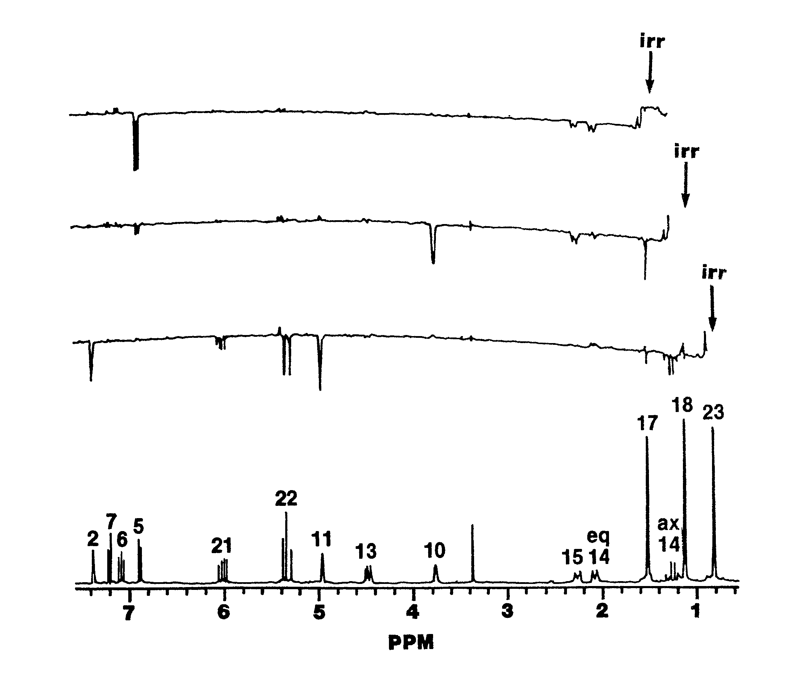

$^1H$ NMR: see the accompanying drawing which is a 300 MHz $^1H$ nuclear magnetic resonance (NMR) spectrum of hapalindole A in dimethyl sulfoxide-$d_6$. The difference nuclear Overhauser effect (nOe) spectra resulting from irradiation of the C-17, C-18, and C-23 methyl groups are also shown (Positive nOes are down).

$^{13}C$ NMR: see Table III

Table III: NMR Data for Hapalindole A (1) in $CDCl_3$

| $^{13}C$ $\delta^{a,b}$ | Carbon | $^1H$ $\delta^c$ |
|---|---|---|
| 157.40 s[d] | 20 | |
| 142.99 d | 21 | 6.100 dd |
| 137.52 s | 8 | |
| 133.17 s | 4 | |
| 123.58 s | 9 | |
| 123.21 d | 6 | 7.190 m |
| 118.48 d | 2 | 6.878 t |
| 115.90 t | 22 | 5.346 dd |
| | | 5.236 dd |
| 113.65 d | 5 | 6.969 m |
| 110.14 s | 3 | |
| 108.33 d | 7 | 7.199 m |
| 63.59 d[d] | 11 | 4.373 br d |
| 62.99 d | 13 | 4.360 dd |
| 44.39 d | 15 | 2.317 ddd |
| 43.86 s | 12 | |
| 37.76 s | 16 | |
| 36.80 d | 10 | 3.875 br m |
| 31.64 q | 18 | 1.193 s |
| 30.82 t | 14 | 2.142[e] dtd |

| $^{13}C\ \delta^{a,b}$ | Carbon | $^{1}H\ \delta^{c}$ |
|---|---|---|
| | | $1.472^{f}$ q |
| 24.07 q | 17 | 1.553 s |
| 18.60 q | 23 | 0.878 s |
| | 1 | 8.085 br |

$J_{H,H}$ (Hz): 1,2 = 2; 1.7 = ∿$0.5^{g}$; 2,6 = ∿$0.5^{g}$; 2,10 = 2; 5,6 = $7.2^{g,h}$; 5,7 = $0.6^{g,h}$; 6,7 = $8.2^{g,h}$; 10,11 = $1.6^{g}$; $10,14_{eq}$ = 1.2; 10,15 = 4.6; $13,14_{ax}$ = 12.4; $13,14_{eq}$ = 4.0; $14_{ax},14_{eq}$ = -13.5; $14_{ax}$,15 = 13.0; $14_{eq}$,15 = 3.8; trans 21,22 = 17.4; cis 21,22 = 10.9; gem 22,22 = 0.5.

---

[a] 75 MHz; $CDCl_3$ as internal reference = 76.90.

[b] $^{1}H$-$^{13}C$ connectivities determined using a phase-cycled 16-step heteronuclear chemical shift correlation map experiment.

[c] 300 MHz; residual $CHCl_3$ as internal reference = 7.25.

[d] Broad 1:1:1 triplet in proton-noise decoupled spectrum, $J_{^{13}C^{14}C}$ ∿ 5 Hz.

[e] Equatorial.

[f] Axial.

[g] Determined in benzene-$d_6$.

[h] From simulation of ABX spectrum shown by protons on C-5, C-6, and C-7.

C. Characteristics of Hapalindole B (2)

Empirical formula: $C_{21}H_{23}N_2ClS$

$[\alpha]_D$: -194° (c 5.1, $CH_2Cl_2$)

IR ($CHCl_3$): 2080, 2160 $cm^{-1}$

High resolution EIMS m/z: 370.1243

$^1H$ NMR ($CDCl_3$): δ 8.064 (br, NH), 7.197 (m, 7.2 and 0.6 Hz, C-5 H), 7.183 (m, 8.2 and 7.2 Hz, C-6 H), 6.961 (m, 8.2 and 0.6 Hz, C-7 H), 6.882 (t, 2.0 Hz, C-2 H), 6.018 (dd, 17.4 and 10.9 Hz, C-21 H), 5.322 (dd, 10.9 and 0.5 Hz, C-22 H cis to C-21 H), 5.123 (dd, 17.4 and 0.5, C-22 H trans to C-21 H), 4.534 (d, 2.3 Hz, C-11 H), 4.319 (dd, 12.6 and 3.9 Hz, C-13 H), 3.867 (br m, C-10 H), 2.220 (ddd, 12.8, 4.6, and 3.6 Hz, C-15 H), 2.149 (dtd, -13.3, 3.9, 3.6, and 1.1 Hz, eq H on C-14), 1.555 (s, 3H on C-17), 1.489 (q, -13.3, 12.8, and 12.6 Hz, ax H on C-14), 1.198 (s, 3H on C-18), 0.870 (s, 3H on C-23). Similar (to 1) nOe effects are seen on irradiation of the Me signals at δ 0.870, 1.198, and 1.555.

$^{13}C$ NMR ($CDCl_3$): δ 143.46 (C-21), 137.78 (C-8), 133.33 (C-4), 132.54 (C-20), 123.83 (C-9), 123.39 (C-6), 118.65 (C-2), 115.74 (C-22), 113.84 (C-7), 110.65 (C-3), 108.45 (C-5), 66.91 (C-11), 63.65 (C-13), 46.04 (C-12), 45.23 (C-15), 38.05 (C-16), 37.49 (C-10), 31.89 (C-18), 31.11 (C-14), 24.31 (C-17), 19.23 (C-23).

D. Characteristics of Hapalindole C (3)

Empirical formula: $C_{21}H_{24}N_2$
mp: 138-143°;
IR ($CHCl_3$): 2150 $cm^{-1}$
$^1H$ NMR ($CDCl_3$): δ 8.115 (br, H on N-1), 7.189 (br d, C-2 H), 7.466 (dm, C-4 H), 7.123 (ddd, C-5 H), 7.185 (ddd, C-6 H), 7.366 (ddd, C-7 H), 3.549 (dd, C-10 H), 3.664 (br m, C-11 H), 2.056 and 1.550 (m, 2H on C-13), 1.83 (m, ax H on C-14), 1.78 (m, eq H on C-14), 2.870 (m, C-15 H), 4.814 (br dq, Z H on C-17), 4.661 (pentuplet, E H on C-17), 1.535 (dd, 3H on C-18), 5.916 (dd, C-21 H), 5.12 (m, E H on C-22), 5.11 (m, Z H on C-22), 1.371 (s, 3H on C-23); J (1,2) = 2.5 Hz, (2,10) = 0.5, (4,5) = 7.7, (4,6) = 1.2, (4,7) = 0.8, (5,6) = 7.1, (5,7) = 1.1, (6,7) = 8.1, (10,11) = 2.8, (10,15) = 12.1, (11,13 eq) = 1.5, (13,13) = -13.9, (13eq,14ax) = 3.8, (14,14) = -13.9, (14ax,15) = 12, (14eq,15) = 3.3, (17,17) = 2.0, (17Z,18) = 0.8, (17E,18) = 1.5, (21, 22Z) = 17.7, (21,22E) = 10.7, (22,22) = 0.9.

E. Characteristics of Hapalindole D [4]

Empirical formula: $C_{21}H_{24}N_2S$
mp: 105-107°
$[\alpha]_D$: + 239° (c 1.2, $CH_2Cl_2$)
IR ($CHCl_3$): 2180, sh 2130 $cm^{-1}$
$^1H$ NMR ($CDCl_3$): δ 8.106 (H on N-1), 7.100 (C-2 H), 7.482 (C-4 H), 7.127 (C-5 H), 7.198 (C-6 H),

7.370 (C-7 H), 3.575 (C-10 H), 3.822 (C-11 H), 1.998 and 1.582 (2H on C-13), 1.84 (ax H on C-14), 1.80 (eq H on C-14), 2.818 (C-15 H), 4.826 (Z H on C-17), 4.666 (E H on C-17), 1.537 (3H on C-18), 5.870 (C-21 H), 5.105 (E H on C-22), 5.095 (Z H on C-22), 1.349 (3H on C-23); coupling constants are essentially the same as above for 3, but signal for H on C-11 does not show coupling to N-19.

F. Characteristics of Hapalindole E [5]

Empirical formula: $C_{21}H_{23}N_2Cl$
mp: 88-90°
$[\alpha]_D$: +25.2° (c 3.1, $CH_2Cl_2$);
IR (KBr): 2140 $cm^{-1}$
High resolution EIMS m/z: 338.1532.

$^1H$ NMR ($CDCl_3$): δ 8.120 (br, H on N-1), 7.168 (br d, C-2 H), 7.439 (ddt, C-4 H), 7.131 (ddd, C-5 H), 7.197 (ddd, C-6 H), 7.377 (ddd, C-7 H), 3.610 (br dd, C-10 H), 3.811 (br d, C-11 H), 4.459 (dd, C-13 H), 2.248 (ddd, eq H on C-14), 2.151 (dt, ax H on C-14), 3.060 (td, C-15 H), 4.852 (dq, Z H on C-17), 4.717 (pentuplet, E H on C-17), 1.548 (dd, 3 H on C-18), 6.045 (dd, C-21 H), 5.299 (dd, E H on C-22), 5.252 (dd, Z H on C-22), 1.481 (s, 3 H on C-23); J (1,2) = 2.2 Hz, (1,10) = 0.3, (4,5) = 7.7, (4,6) = 1.2, (4,7) = 0.8, (5,6) = 7.1, (5,7) = 1.1, (6,7) = 8.1, (10,11) = 2.9, (10,15) = 12.1, (13,14ax) = 12.1, (13,14eq) = 5.0, (14ax,14eq) = -13.7, (14ax,15) = 12.1, (14eq,15) = 4.3, (17,17) = 1.5,

(17$\underline{Z}$,18) = 0.8, (17$\underline{E}$,18) = 1.5, (21,22$\underline{E}$) = 10.9, (21,22$\underline{Z}$) = 17.5, (22,22) = 0.3.

$^{1}$H(irr)→$^{1}$H(nOe): δ 3.610 → 7.439(+), 3.811(+); 3.060 → 4.852(+), 4.459(+); 1.548 → 4.717(+), 3.610(+), 2.151(+); 1.481 → 5.252(+), 3.811(+), 3.610(+) 2.151(+).

$^{13}$C NMR ($CDCl_3$): δ 123.45 (C-2), 111.74 (C-3), 116.84 (C-4), 119.52 (C-5), 122.05 (C-6), 111.44 (C-7), 135.50 (C-8), 126.23 (C-9), 34.71 (C-10), 67.04 (C-11), 44.54 (C-12), 60.77 (C-13), 38.10 (C-14), 43.90 (C-15), 145.10 (C-16), 113.36 (C-17), 18.55 (C-18), 158.45 (C-20), 141.70 (C-21), 116.06 (C-22), 16.04 (C-23).

G. Characteristics of Hapalindole F ($\underline{6}$)

Empirical formula: $C_{21}H_{23}N_2ClS$
mp: 176-179°
IR (KBr): 2170, 2110 $cm^{-1}$
$^{1}$H NMR ($CDCl_3$): δ 8.149 (H on N-1), 7.083 (C-2 H), 7.465 (C-4 H), 7.139 (C-5 H), 7.210 (C-6 H), 7.378 (C-7 H), 3.649 (C-10 H), 4.026 (C-11 H), 4.411 (C-13 H), 2.256 (eq H on C-14), 2.170 (ax H on C-14), 3.018 (C-15 H), 4.866 ($\underline{Z}$ H on C-17), 4.724 ($\underline{E}$ H on C-17), 1.550 (3 H on C-18), 5.984 (H on C-21), 5.277 ($\underline{E}$ H on C-22), 5.233 ($\underline{Z}$ H on C-22), 1.467 (3H on C-23); coupling constants are essentially the same as those above for $\underline{5}$.

H. Characteristics of Hapalindole G (7)

Empirical formula: $C_{21}H_{23}N_2Cl$
mp: >185°C (dec)
IR($CHCl_3$): 2150 $cm^{-1}$
High resolution EIMS: m/z 338.153

$^1$H NMR ($CDCl_3$): δ 8.044 (br, H on N-1), 7.197 (m, C-7 H), 7.184 (m, C-6 H), 7.035 (m, C-5 H), 6.891 (dd, C-2 H), 6.137 (dd, C-21 H), 5.394 (dd, E H on C-22), 5.342 (dd, Z H on C-22), 4.430 (dd, C-13 H), 4.238 (br d, C-11 H), 3.322 (br dm, C-10 H), 2.407 (ddd, eq H on C-14), 2.105 (ddd, C-15 H), 2.005 (dt, ax H on C-14), 1.520 (s, 3H on pseudo eq C-18), 1.388 (s, 3H on ax C-23), 1.168 (s, 3H on pseudo ax C-17); J(1,2) = 2.2 Hz, J(2,10) = 1.6, J(5,6) = 7.2, J(5,7) = 0.6, J(6,7) = 8.2, J(10,11) = 3.1, J(10,15) = 10.4, J(13,14eq) = 4.5, J(13,14ax) = 11.9, J(14eq,14ax) = -12.5, J(14eq,15) = 3.0, J(14ax,15) = 11.9, J(21,22Z) = 17.4, J(21,22E) = 10.9, J(22,22) = 0.5.

$^1$H(irr) → $^1$H(nOe): 1.520 → 7.035(+), 2.407(+), 2.105(+); 1.388 → 6.137 (small +), 5.342(+), 4.238(+), 3.322(+), 2.005(+); 1.168 → 7.035 (small +), 3.322(+), 2.005(+).

I. Characteristics of Hapalindole H (8)

Empirical formula: $C_{21}H_{24}N_2$
mp: 190-193°
$[\alpha]_D$: + 152° (c 4.1, $CH_2Cl_2$)

IR(KBr): 2150 $cm^{-1}$

EIMS: m/z 304

$^1H$ NMR ($CDCl_3$): δ 8.038 (br, H on N-1), 7.621 (dd, C-2 H), 7.020 (m, C-5 H), 7.176 (m, C-6 H), 7.189 (m, C-7 H), 3.167 (td, C-10 H), 3.505 (br dtd), 2.066 (dt, eq H on C-13), 1.380 (td, ax H on C-13), 1.785 (dq, eq H on C-14), 1.657 (qd, ax H on C-14), 1.517 (ddd, C-15 H), 1.449 (s, 3H on C-17), 1.106 (s, 3H on C-18), 6.288 (ddd, C-21 H), 5.358 (ddd, E H on C-22), 5.290 (dd, Z H on C-22), 1.278 (s, 3 H on C-23); J(1,2) = 2.2 Hz, (2,10) = 1.6, (5,6) = 7.2, (5,7) = 0.6, (6,7) = 8.2, (10,11) = 10.8, (10,15) = 11.0, (11,22 E) = 0.4, (13,13) = -13.9, (13ax, 14ax) = 13, (13ax,14eq) = 3.5, (13eq, 14ax) = 3.2, (13eq,14eq) = 3.5, (13ax,21) = 0.5, (14,14) = -13.2, (14ax,15) = 12.0, (14eq,15) = 3.5, (21,22E) = 11.1, (21,22Z) = 17.5, (22,22) = 1.1

$^1H$(irr) → $^1H$(nOe): δ 1.449 → 7.020(+); 1.278 → 6.288(+), 5.290(+), 3.505(+); 1.106 → 3.167(+)

$^{13}C$ NMR ($CDCl_3$): δ 118.33 (C-2), 113.03 (C-3), 140.54 (C-4), 112.47 (C-5), 108.07 (C-6), 122.56 (C-7), 133.22 (C-8), 124.88 (C-9), 36.24 (C-10), 67.83 (C-11), 37.25 (C-16), 36.14 (C-13), 20.78 (C-14), 49.73 (C-15), 40.47 (C-12), 24.47 (C-17), 24.75 (C-18), 157.53 (C-20), 138.47 (C-21), 115.83 (C-22), 27.24 (C-23).

J. Characteristics of Hapalindole I (9)

Empirical formula: $C_{21}H_{21}N_2Cl$

mp: 180° (dec)

IR (KBr): 2110 $cm^{-1}$

High resolution EIMS: m/z 336.1366

$^{1}H$ NMR ($CDCl_3$): δ 8.427 (br, H on N-1), 7.903 (d, C-2 H), 7.061 (m, C-5 H), 7.239 and 7.226 (m, C-6 H and C-7 H), 4.140 (dd, C-13 H), 2.405 (ddd, eq H on C-14), 2.225 (td, ax H on C-14), 2.860 (dd, C-15 H), 1.542 (s, 3 H on C-17), 1.064 (s, 3H on C-18), 5.850 (dd, C-21 H), 5.459 (dd, E H on C-22), 5.358 (dd, Z H on C-22), 1.473 (s, 3 H on C-23); J(1,2) = 2.6, (5,6) = 7.2, (5,7) = 0.6, (6,7) = 8.2, (13,14ax) = 12.9, (13,14eq) = 3.6, (14ax,15) = 11.6, (14eq,15) 6.2, (14,14) = -13.2, (21,22 E) = 10.7, (21,22 Z) = 17.3, (22E,22Z) = 0.5

$^{1}H$(irr) → $^{1}H$(nOe): δ 1.542 → 7.061(+), 2.860(+), 2.405(+); 1.473 → 5.850(+), 5.358(+), 2.225(+); 1.064 → 2.225(+).

CLAIMS

1. A process for preparing hapalindole A, B, C, D, E, F, G, H or I, as hereinbefore defined, which comprises culturing a strain of Hapalosiphon fontinalis which produces these compounds under submerged aerobic conditions in a suitable culture medium until substantial antibiotic activity is produced.

2. A process according to claim 1 wherein the strain is Hapalosiphon fontinalis ATCC 39694.

3. Hapalindole A of the formula:

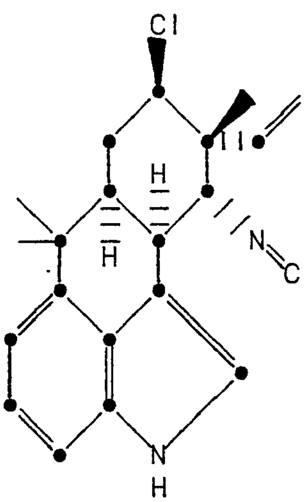

4. Hapalindole B of the formula

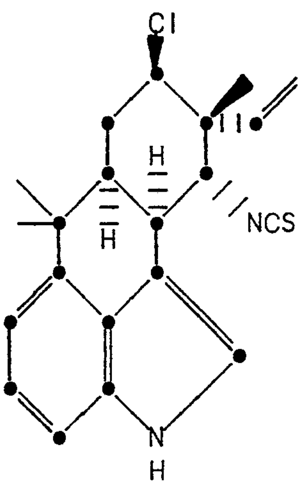

5. A compound of the formula

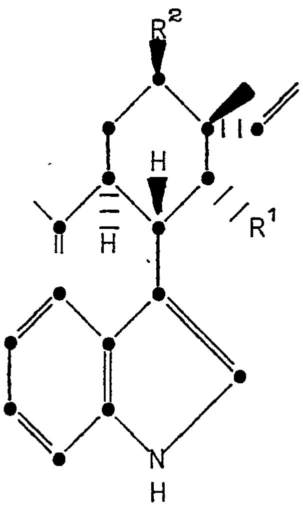

wherein $R^1$ is -NC or -NCS and $R^2$ is hydrogen or chlorine.

6. The compound of claim 5 wherein $R^1$ is -NC and $R^2$ is hydrogen.

7. The compound of claim 5 wherein $R^1$ is -NC and $R^2$ is chlorine.

8. Hapalindole G of the formula

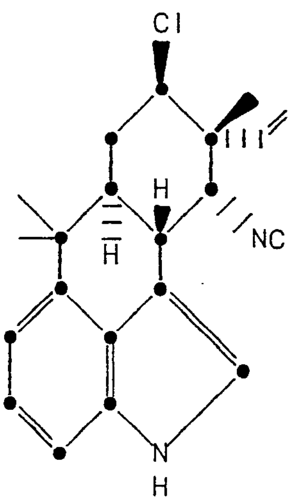

9. Hapalindole H of the formula

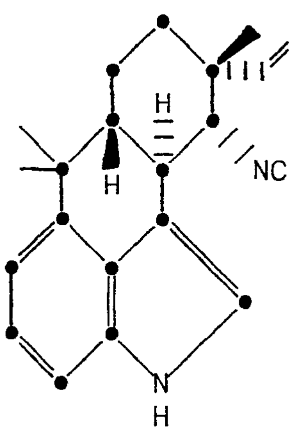

10. Hapalindole I of the formula:

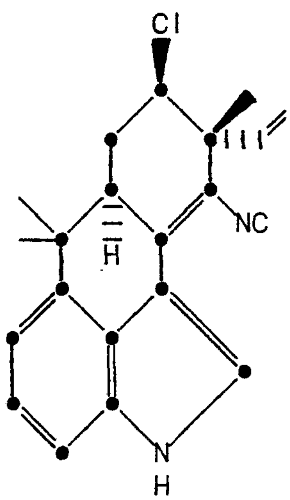

11. A pharmaceutical formulation which comprises as an active ingredient hapalindole A, B, C, D, E, F, G, H or I, as hereinbefore defined, associated with one or more pharmaceutically-acceptable carriers therefor.

12. Hapalindole A, B, C, D, E, F, G, H or I for use as a pharmaceutical.

## CLAIMS

1. A process for preparing hapalindole A, B, C, D, E, F, G, H or I, as hereinbefore defined, which comprises culturing a strain of *Hapalosiphon fontinalis* which produces these compounds under submerged aerobic conditions in a suitable culture medium until substantial antibiotic activity is produced.

2. A process according to claim 1 wherein the strain is Hapalosiphon fontinalis ATCC 39694.

0171283